# EUROPEAN PATENT APPLICATION

(11) **EP 4 283 627 A1**
(43) Date of publication of application: **29.11.2023**
(21) Application number: 22175186.0
(22) Date of filing: 24.05.2022
(51) Int. Cl.: G16H 20/40, G16H 50/20

(54) **METHOD FOR USE IN AN ORAL CARE SYSTEM**

(71) Applicant: Koninklijke Philips N.V., 5656 AG Eindhoven (NL)
(72) Inventor: VAN LEEUWEN, Marinus, Bastiaan, Eindhoven (NL); YUAN, Zhaorui, Eindhoven (NL)
(74) Representative: Philips Intellectual Property & Standards

(57) **Abstract**

A teledentistry method and system facilitating communication of oral care advice, and means for objectively analyzing adherence by patients to issued advice through standardization of oral status input by a dentist, standardized conversion from oral status to advice and conversion of advice to sensor-detectable parameters to enable compliance monitoring.

## Description

### FIELD OF THE INVENTION

This invention relates generally to the field of oral care, and in particular to the field of teledentistry.

### BACKGROUND OF THE INVENTION

A typical oral healthcare workflow for a patient comprises a combination of oral cleaning at home and regular check-ups by a dentist and/or oral hygienist. The vast majority of oral cleaning and maintenance of the patient takes place at home. During the regular (e.g. bi-yearly) visits to a dental practitioner, a professional assesses the oral health status of the patient, applies immediate treatment if necessary (e.g. filling of caries, removal of calculus/tartar), and provides advice as to how to improve oral care at home. This advice is typically given verbally, or in written form.

This standard approach has many weaknesses, including at least the following. First, the advice given by the professional might be forgotten by the patient (either immediately or over time). Second, the advice given by the professional might be misunderstood by the patient (completely or partially). Third, there is no means for reliably providing feedback to a dental practitioner as to how well the advice is being implemented at home, during the period between the regular check-ups.

Consequently, the oral cleaning between the regular check-ups can be sub-optimal, resulting in sub-optimal oral hygiene. Sub-optimal oral care typically results in sub-optimal systemic healthcare, discomfort for the patient, deterioration of existing oral problems (e.g. the developing of gingivitis), and increased cost of (oral and general) healthcare.

Developments in this field, able to address one or more of the above problems, would be of value.

### SUMMARY OF THE INVENTION

The invention is defined by the claims.

According to examples in accordance with an aspect of the invention, there is provided a method for facilitating interaction between a primary user and a secondary user of an oral care system. The primary user may be a user of an oral care device (i.e. the patient in the previously discussed scenario) and the secondary user may be a dental care professional. The method comprises receiving as user input from the secondary user, at an input device at a first location, oral examination findings corresponding to the primary user, wherein the findings comprise user-input values for a pre-defined set of standardized oral parameters which together define an examination finding set. The method further comprises applying an advice generation algorithm adapted to convert an examination finding set to oral care advice comprising one or more oral care advice items, the advice generation algorithm defining mappings between different possible values for the pre-defined set of standardized oral parameters and output oral care advice items. The method further comprises uploading the oral care advice to a record in a user database, the record associated with the identity of the primary user. Each oral care advice item preferably includes an encoded representation of acceptable parameter values for one or more oral cleaning parameters.

The method further comprises, at a second location: an oral care device obtaining sensor data during one or more oral cleaning sessions of the primary user from sensors integrated in the oral care device; applying a sensor data conversion algorithm for converting the sensor data to an estimation of values for the one or more oral cleaning parameters for at least one oral cleaning session; comparing the estimated values for the one or more oral cleaning parameters with the acceptable parameter values encoded in the oral care advice; and generating an output based on the comparison.

Thus, the method has parts that take place at a first location and parts that take place at a second location, where the first location may be a dental clinical environment for example, and the second location may be a home of the primary user (the patient).

There are, in effect, three key elements embodied by this method: first, the standardization of the input findings of the dentist and the conversion of these findings to template advice items matched to the findings; second, the advice items encoding cleaning parameters which are intelligible and detectable by an oral care device; and third, the measuring of the oral care parameters during use of the oral care device and the comparison with the advised parameters. By standardizing the input findings of the dentist, this facilitates the automated conversion into advice items. By having the advice items relate to well-defined and measurable oral care parameters, this facilitates the automatic detection and monitoring at the second location of the adherence to the advice in a way not possible in the traditional oral health monitoring workflow.

Embodiments of the invention have been motivated by an aim to remove the previously discussed weaknesses in the traditional oral health care workflow. The proposed method improves the efficiency of the oral care process, and improves the effectiveness of the regular visits, resulting in improved oral health and lower cost of healthcare. It also provides reassurance to the patient that his or her cleaning behavior is good (in a seamless fashion and without adding significant overhead).

In some embodiments, the application of the sensor data conversion algorithm and/or the comparing of the estimated values for the cleaning parameters is performed by a processor of the oral care device. In some other embodiments, this is done by a processor of a computing device communicably linked with the oral care device. This may for example be a mobile computing device, e.g. a smartphone of a user.

In some embodiments, the one or more oral care advice items output from the advice generation algorithm are presented to the secondary user via a user interface, and wherein the secondary user is prompted to confirm, reject, or refine the one or more advice items using the user interface. In other words, the secondary user (who may be dental practitioner inputting the findings) is presented with a draft version of the advice which will be issued to the primary user (the patient) and can review before finalization.

The method may comprise a computing device of the primary user, or the oral care device, establishing communication with the - user database responsive and downloading the oral care advice. This may be user-triggered or automatically triggered. If a computing device (e.g. mobile computing device) does this, the advice may optionally further be communicated to the oral care device.

In some embodiments, the method comprises, responsive to a user-activation of the oral care device, transmitting a notification signal to a computing device, the computing device establishing communication with the user database responsive to the activation signal and downloading the oral care advice. Again, this computing device may be a mobile computing device.

Thus, advantageously, the advice items are automatically downloaded to a device of the primary user upon switching on the oral care device, avoiding that the user might otherwise forget.

In some embodiments, the generated output may include a user-perceptible output. The generated output may additionally or alternatively comprise a data output indicative of a result of the comparison.

In some cases, a user-perceptible output may be generated at the computing device or the oral care device indicative of the oral care advice.

In some embodiments, the oral care device is a powered toothbrush, and the previously mentioned sensors include one or more of: pressure sensors for sensing brushing pressure, an inertial measurement unit (IMU) for use in determining a pose of the oral care device, and one or more optical sensors for optical interaction with oral surfaces during use of the device. Pose can for example be determined using a combination of acceleration and angular velocity measurements of the IMU.

In some embodiments, the method further comprises presenting at said input device (at the first location) a graphical user interface, the graphical user interface defining discrete input fields, each corresponding to one of the standardized oral parameters, and wherein the oral examination findings are received as user input to the graphical user interface. This advantageously provides an efficient means for facilitating the input of standardized findings. By providing only restricted fields into which findings can be input, this ensures the input data is in a form, and pertains to a set of fields, that is suitable for application of the advice generation algorithm.

In some embodiments, the method comprises generating a user compliance report based on the comparison of the estimated values for the one or more oral cleaning parameters with the acceptable parameter values encoded in the oral care advice, the report indicative of a degree of compliance of the primary user with the oral care advice. This may be transmitted as data to a further device. For example, the oral care device or the computing device of the primary user may generate the compliance report. The method may further comprise uploading the compliance report to a server and storing it at the server in such a way as to be accessible by the secondary user via a computing device communicatively linked to the server. Thus, this advantageously facilitates feedback to a dental practitioner as to how well a user is following the generated advice.

The compliance report may also be communicated to a medical insurance company in some examples.

In some embodiments, following the generating of the oral care advice, the oral care advice is uploaded to a digital patient record associated with the primary user in a patient record database. The digital patient record may also store further clinically relevant information about the primary user, for instance personal details, as well as clinical/medical history including dental health history. Thus, the advice can efficiently be stored in a single location with the rest of the patient's clinical data.

In some embodiments, a user-perceptible output indicative of the oral care advice is generated. This may be generated in some embodiments by the oral care device, e.g. by illumination of a subset of one or more LEDs comprised by the device, to provide a coded indication of the advice, or by control of an acoustic output device of the oral care device to play a spoken-language indication of the advice. Other components or devices could additionally or alternatively be used to generate the user-perceptible output indicative of the oral care advice, such as, by way of non-limiting example, a charging station of the toothbrush, or a mobile computing device.

In some examples, the user-detectable output may be generated repeatedly until a user-operable control is actuated by a user. Thus, not only is the encoded advice automatically compared with cleaning performance, but the user is informed of the advice in a form which is understandable, so that they can actively seek to match their cleaning behavior to the advice.

In some embodiments, the one or more oral care advice items output from the advice generation algorithm may be items from a pre-determined list of oral care advice items. In other words, the advice generation algorithm maps input findings to a set of pre-determined advice items, rather than creatively generating new advice items, although the latter is also an option using for instance artificial intelligence solutions.

In some embodiments, advice generation algorithm comprises a lookup table which maps between findings and advice items.

Above has been described embodiments of the invention in the form of a method. The invention can also be embodied in a computer program product comprising code means. The code means may be configured when executed by a processor to perform either one of two methods according to a selected mode. For example, the first method may be for execution in association with a dental practitioner (a secondary user) in a clinical environment, and the second mode may be for performing by a patient (a primary user) at home.

The method according to a first mode comprises:
receiving as user input from the secondary user at an input device oral examination findings corresponding to the primary user, wherein the findings comprise user-input values for a pre-defined set of standardized oral parameters which together define an examination finding set;
applying an advice generation algorithm adapted to convert an examination finding set to oral care advice comprising one or more oral care advice items, the advice generation algorithm defining mappings between different possible values for the pre-defined set of standardized oral parameters and output advice items;
uploading the oral care advice to a record in a user database, the record associated with the identity of the primary user;
wherein each oral care advice item includes an encoded representation of acceptable parameter values for one or more oral cleaning parameters.

The method according to a second mode, comprises:
receiving sensor data obtained by an oral care device during one or more oral cleaning sessions of the primary user from sensors integrated in the oral care device,
applying a sensor data conversion algorithm for converting the sensor data to an estimation of values for the one or more oral cleaning parameters for at least one oral cleaning session,
comparing the estimated values for the one or more oral cleaning parameters with the acceptable parameter values encoded in the oral care advice, and
generating an output based on the comparison.

For example, the first mode may be implemented when the application is run on a computing device or user-input device at a dentist's office.

For example, the second mode might be implemented when the application is run on a mobile computing device of the primary user communicatively linked with the oral care device, or by the oral care device itself.

Thus, conveniently, a same software application can be used to perform both the clinician-side and patient-side of the communication method. For example, a user might be prompted to login to the app, and wherein the user account defines whether the app is to be run in clinician mode for that user (first mode) or patient mode for that user (second mode).

The invention can also be embodied in the form of apparatus.

For example, one aspect of the invention is a system for use in facilitating interaction between a primary user and a secondary user, wherein the primary user is a user of an oral care device and the secondary user is a dental care professional,

The system comprises.
a first device (e.g. at a first location) comprising one or more processors and adapted to:
receive as user input from a secondary user an input device oral examination findings corresponding to the primary user, wherein the findings comprise user-input values for a pre-defined set of standardized oral parameters which together define an examination finding set;
apply an advice generation algorithm adapted to convert an examination finding set to oral care advice comprising one or more oral care advice items, the advice generation algorithm defining mappings between different possible values for the pre-defined set of standardized oral parameters to output oral care advice items;
upload the oral care advice to a record in a user database, the record associated with the identity of the primary user;
wherein each oral care advice item includes an encoded representation of acceptable parameter values for one or more oral cleaning parameters.

The system further comprises a second device (e.g. at a second location), comprising one or more processors adapted to:
receive sensor data obtained by an oral care device during one or more oral cleaning sessions of the primary user from sensors integrated in the oral care device,
apply a sensor data conversion algorithm for converting the sensor data to an estimation of values for the one or more oral cleaning parameters for at least one oral cleaning session,
compare the estimated values for the one or more oral cleaning parameters with the acceptable parameter values encoded in the oral care advice, and
generate an output based on the comparison.

In some embodiments, the second device may be the oral care device or may be a mobile computing device communicatively linked with the oral care device.

The oral care device may be a powered toothbrush.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of steps of a method in accordance with one or more embodiments of the invention; and
Fig. 2 is a schematic block diagram of a system in accordance with one or more embodiments of the invention.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a teledentistry method and system facilitating communication of oral care advice and means for objectively analyzing adherence by patients to issued advice through standardization of oral status input by a dentist, standardized conversion from oral status to advice and conversion of advice to sensor-detectable parameters to enable compliance monitoring.

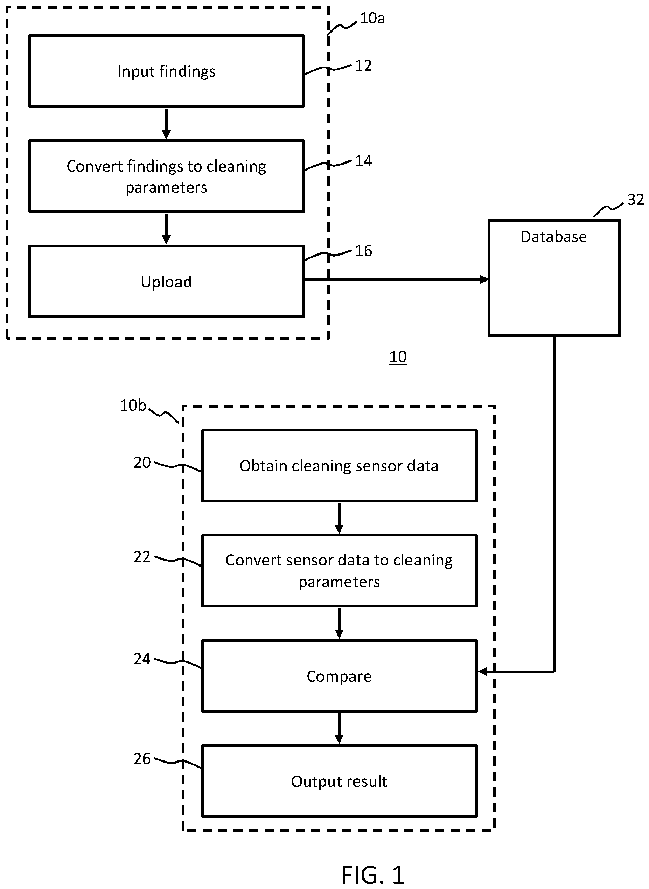

Fig. 1 outlines in block diagram form steps of an example method according to one or more embodiments. The steps will be recited in summary, before being explained further in the form of example embodiments. The method may be computer implemented.

By way of overview, the method 10 is for facilitating interaction between a primary user and a secondary user of an oral care system, wherein the primary user is a user of an oral care device, and the secondary user is a dental care professional.

The method can be understood as comprising two parts 10a, 10b, wherein a first part 10a of the method is done at a first time, and at a first location (e.g. during a dental examination at a dental practice), and the second part 10b of the method being done at a second time, or more likely a plurality of second occasions, at a second location (e.g. at a patient's home, each time they perform oral care such as cleaning their teeth).

The first part of the method (at the dental practice) is as follows.

The method comprises receiving 12 as user input from the secondary user (e.g. the dentist), at an input device at a first location, oral examination findings corresponding to the primary user, wherein the findings comprise user-input values for a pre-defined set of standardized oral parameters which together define an examination finding set. For example, the input device may be a computing device, e.g. a personal computer, or a mobile computing device such as a smartphone. The findings might include for instance oral parameters such as an amount of plaque/calculus detected per tooth, presence of any caries, status of gums at different oral sites, tooth wobble amount, and/or any other clinically relevant findings. By 'standardized' oral parameters is meant that there are a pre-defined finite set of different parameters that the secondary user can provide input in relation to, and each of these may allow only a certain type of entry (e.g. binary, continuous numeric value optionally limited to a certain range, a finite list of possible quantitative or qualitative values and so on). In this way, the input findings are constrained to conform to a standardized set of oral parameters.

The method further comprises applying 14 an advice generation algorithm adapted to convert an examination finding set to oral care advice comprising one or more oral care advice items. The advice generation algorithm for example defines mappings between different possible values for the pre-defined set of standardized oral parameters and output oral care advice items. In this way, there is standardization and reproducibility in the conversion of the oral parameters to the oral care advice items. The one or more oral care advice items output from the advice generation algorithm may be items from a pre-determined list of oral care advice items. In other words, the advice items are finite and constrained in terms of the type and quality of the advice which is issued, which allows for more straightforward conversion, and ensures reproducibility.

In a preferred embodiment, each oral care advice item includes an encoded representation of acceptable parameter values for one or more oral cleaning parameters. As will become clear when the second part of the method 10b is explained, this advantageously means that adherence to the advice is directly testable via measurements taken during oral cleaning by the user. In other words, the oral cleaning parameters define parameters or properties of oral cleaning/care actions to be taken by the primary user which are testable/measurable, preferably through automatically acquired sensor data so that the testability is objective. The oral care advice items may include additional advice items beyond the acceptable parameter values for the one or more oral cleaning parameters. This might be more qualitative advice targeted at the user, for example about diet (e.g. avoid sugary foods at certain times, avoid carbonated beverages, reminder not to grind teeth etc.).

To ensure reproducibility of the conversion, the advice generation algorithm may comprise a lookup table which defines mappings between input findings and output advice items.

The method further comprises uploading 16 the oral care advice to a record in a user database 32, the record associated with the identity of the primary user. This database is typically stored on a remotely located datastore, for instance a server. The database may be a cloud-based database; in other words a database which is stored at a server which permits access the database via an internet or even a private network link to one or more credentialed users, for example the primary user and the secondary user.

The second part of the method 10b (taking place most likely at another location) is as follows.

The method comprises an oral care device obtaining 20 sensor data during one or more oral cleaning sessions of the primary user from one or more sensors integrated in the oral care device. The sensor data may for example be data indicative of handling of the oral care device, or engagement patterns between the oral care device and oral surfaces, of positioning of the device within the mouth and relative to oral surfaces, and so on. By way of illustration, the one or more sensors may include for example pressure sensors for sensing brushing pressure (in the event the oral care device is a toothbrush), an inertial measurement unit (IMU), e.g. for use in determining a pose of the oral care device, and/or one or more optical sensors for optical interaction with oral surfaces during use of the device. For instance, with reference to the IMU, this might measure acceleration and angular velocity. These two together can be used to derive pose estimation for the brush. Analysis of the IMU data as a function of time allows for localization of the toothbrush in the mouth over the cleaning session. In summary, the sensor data aims to characterize each oral care session performed by the user using the oral care device.

The method further comprises applying 22 a sensor data conversion algorithm for converting the sensor data to an estimation of values for the one or more oral cleaning parameters for at least one oral cleaning session. As already explained, the benefit of generating advice items which encode oral cleaning parameters is that it allows objective testing through converting sensor to values for the same standardized set of oral cleaning parameters. The method further comprises comparing 24 the estimated values for the one or more oral cleaning parameters with the acceptable parameter values encoded in the oral care advice. The method further comprises generating 26 an output based on the comparison. The output may include a user-perceptible output. By way of example, the user-perceptible output indicative of the oral care advice might be generated by the oral care device, e.g. by illumination of a subset of one or more LEDs comprised by the device, to provide a coded indication of the advice, or by control of an acoustic output device of the oral care device to play a spoken-language indication of the advice. A user perceptible output can additionally or alternatively be generated by another device, e.g. a charging station of the toothbrush, or a mobile computing device.

Optionally, in accordance with some embodiments, the method may further comprise communicating with a user registration database which stores a registration record associated with the primary user, said registration record indicating a permission level of the primary user. The permission level may define a set or range of functionalities which are accessible to the primary user. For example, the permission level may define a subset of the oral cleaning parameters which the oral care device of the primary user will measure and/or which will be compared (by whichever device performs the comparison) with the acceptable parameter values included in the advice items. By way of example, a user permission level may vary depending upon a subscription package to which the user is registered, and with a higher level of subscription 'unlocking' permissions for a greater range of oral cleaning parameters which will be monitored and/or compared against acceptable parameter values encoded in the advice items.

Similarly, the registration database may additionally or alternatively store a record associated with the secondary user, this recording a permission level of the secondary user. The permission level may define a subset or sub-range of the standardized oral parameters which the secondary user is able to provide input-values in relation to, and/or a subset or sub-range of the output oral care advice items which can be generated, and/or a subset or sub-range of the one or more oral cleaning parameters acceptable parameter values for which can be issued in the name of the secondary user to any primary user. The permission level of the secondary user may vary in accordance with a subscription level of the user recorded in the registration database, with a higher subscription package 'unlocking' additional of any of the aforementioned parameters or advice items.

In some embodiments, a permission level of the primary user may be determined in advance of the secondary user inputting at the input device the oral examination findings, or concurrently with this step. This may allow the system facilitating the input to restrict input of the secondary user to only those oral parameters for which the permission level of the primary user will permit monitoring via associated oral cleaning parameters available to the primary user.

Additionally or alternatively, in some embodiments, the registration record of the primary user may define a subset of sensing functionalities which are available to the primary user in the oral care device, and these may thereby restrict the range of oral cleaning parameters which can be measured when using the oral care device. In a similar fashion as described above, this permission level may be defined according to a subscription package of the primary user, with higher subscription levels unlocking additional sensor functionalities, and thereby permitting the user to have a broader range of oral cleaning parameters monitored and thus for adherence to a broader range of advice items to be monitored.

The method may additionally comprise an explicit step of downloading the oral care advice from the database 32, and this might be performed by a computing device of the primary user, for example (but not necessarily) a mobile computing device. In one set of embodiments for instance, the method further comprises, responsive to a user-activation of the oral care device, transmitting a notification signal to a computing device, the computing device establishing communication with the central user database responsive to the activation signal and downloading the oral care advice and optionally also generating a user-perceptible output at the computing device or the oral care device indicative of the oral care advice. For example, the oral care device may transmit the notification signal to a mobile computing device responsive to the user activation of the device, the mobile computing device may establish communication with the central user database responsive to the activation signal and download the oral care advice. The optional user-perceptible output may be generated at the mobile computing device or the oral care device.

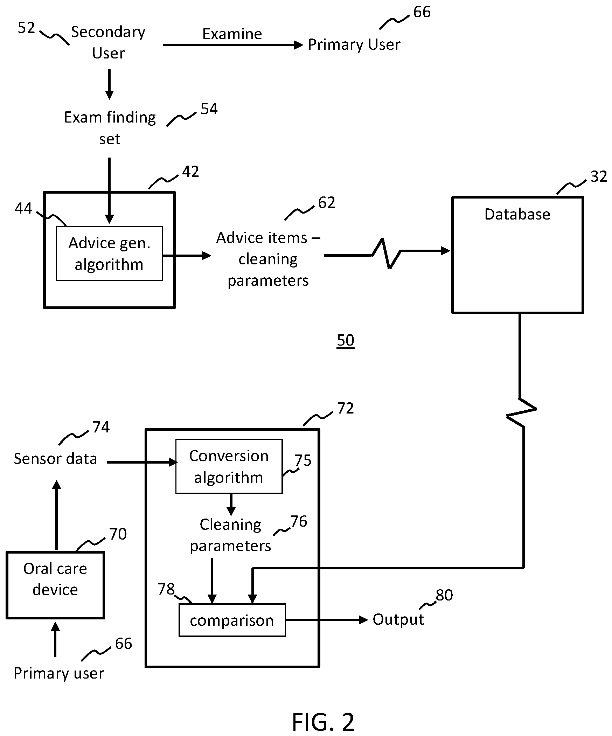

As will be more apparent with the forthcoming description of Fig. 2, there are different options for the apparatus which executes the various steps. For example the conversion 22 and comparison 24 steps might both be performed by a computing device which is communicatively linked with an oral device which obtains 20 the sensor data. Indeed, obtaining the sensor data may in fact be a step of receiving a dataset already acquired by another device, and thus the data obtaining step 20 could also be performed by the same computing device which performs steps 22-24. In other examples, steps 20-24 could all be performed by the oral care device, using a built-in processor and communication module.

In addition to or instead of the user-perceptible output, the output may include a data output which represents the result of the comparison (and thus represents compliance with the advice). This data output may be communicated to the central database for later access by the secondary user, or may be directly communicated to the secondary user, e.g. as an attachment in an email or through a mutually accessible online portal for instance. In some embodiments, the method may comprise generating a user compliance report based on the comparison 24, indicative of a degree of compliance of the primary user with the oral care advice. This may be transmitted to a further device as a data set. For example, it may be uploaded to a server and stored at the server in such a way as to be accessible by the secondary user via a computing device communicatively linked to the server.

The above-outlined method represents an embodiment (or set of embodiments) of the invention which can be fully computer implemented. However, extensions to the method can also be considered which also embody the inventive concept and involve certain steps performed by the primary or secondary user, such as the data entry by the secondary user, the activation of the oral care device and its operation by the primary user and so on.

Fig. 2 shows an example system which is in accordance with one or more embodiments of the invention. The system reflects an example apparatus for performing the method already described in detail above. The parts of the system will be outlined in summary, and it should be understood that the various features and options described above in relation to the methodological embodiments of the inventive concept can be embodied also in examples of the described system.

By way of introduction, the system 50 is for use in facilitating interaction between a primary user 66 and a secondary user 52, wherein the primary user is a user of an oral care device 70 and the secondary user is a dental care professional.

The system comprises a first device 42 at a first location comprising one or more processors. This is a computing device and may be a terminal-based computing device or a mobile computing device by way of two examples. The first device 42 is adapted to receive as user input from a secondary user 52 at a user interface of the first device oral examination findings 54 corresponding to the primary user 66, wherein the findings comprise user-input values for a pre-defined set of standardized oral parameters which together define an examination finding set. The first device 42 is further adapted to apply an advice generation algorithm 44 adapted to convert an examination finding set 54 to oral care advice 62 comprising one or more oral care advice items, the advice generation algorithm defining mappings between different possible values for the pre-defined set of standardized oral parameters 54 to output oral care advice items 62. As explained above, in a preferred embodiment, each oral care advice item includes an encoded representation of acceptable parameter values for one or more oral cleaning parameters. The first device 42 is further adapted to upload the oral care advice 62 to a record in a central user database 32, the record associated with the identity of the primary user 66.

The system further comprises a second device 72, comprising one or more processors. The second device is adapted to receive sensor data obtained by an oral care device 70 during one or more oral cleaning sessions of the primary user 66 from sensors integrated in the oral care device. The system may include the oral care device in some embodiments, but this is not essential. Indeed, the second device 72 could be the oral care device 70 itself in some embodiments, so that there are not two devices 70, 72, but just one, in the form of the oral care device 70.

The second device 72 is further adapted to apply a sensor data conversion algorithm 75 for converting the sensor data 74 to an estimation of values for the one or more oral cleaning parameters 76 for at least one oral cleaning session. The second device 72 is further adapted to compare 78 the estimated values for the one or more oral cleaning parameters with the acceptable parameter values encoded in the oral care advice 62. The second device is further adapted to generate an output 80 based on the comparison.

The second device 72 is a computing device. It may comprise a mobile computing device but this is not essential. The second device 72 is communicatively linked with the oral care device 70 to facilitate transfer of the sensor data 74 and/or other data.

Although all of the steps performed at the second location were described as being performed by the same second device, they could be performed by two or more different devices in mutual communication with one another, or they could all be performed by the oral care device 70 itself.

An example implementation of the method according to a particular set of embodiments will now be described by way of illustration of the above-summarized concept of the invention. It will be appreciated that not all features of this particular set of embodiments are essential to the inventive concept and are described to aid understanding and to provide an example to illustrate the inventive concepts.

In accordance with this particular set of embodiments, the system includes an oral care device 70 in the form of a powered toothbrush. The toothbrush includes an IMU motion sensor. The system includes a mobile computing device having a software app installed thereon for performing some or all of the steps of the second part 10b of the method 10 already described. Each of the toothbrush and the mobile computing device are equipped with a wireless communication module for facilitating wireless communication between these two devices. This could for example be a direct Bluetooth connection, or connection via a wireless network link (e.g. Wi-Fi).

The toothbrush may further include a user-perceptible output means, such as a set of one or more LEDs for providing an optical output, and/or a speaker for providing an acoustic output.

The system further includes a data entry means at the dental office of the dentist. This is a computing device (mobile or otherwise), and this also has a software application installed which facilitates performing of some or all of the steps of the first part 10a of the method 10 already described. Advantageously the software app used by the primary user (of the toothbrush) and the secondary user (the dentist) may be the same software app, but with two different operation modes (clinician vs patient), and wherein login with user credentials for instance determines which mode the application operates in, and where the first mode leads to execution of the first part 10a of the method already described and the second mode leads to execution of the second part 10b of the method already described.

The operation flow of the system and method in accordance with this particular set of embodiments may be as follows.

The patient (the primary user 66) visits the dental office for a regular check-up. The dental professional (the secondary user 52), e.g. dentist or oral hygienist, assesses the status of the oral health of the patient.

From the assessment, observations of the professional are (semi-) automatically converted into one or more advice items from a limited set of advice items concerning how to improve oral cleaning habits. The conversion result can sometimes be a single advice item, a combination of several advice items, or a subset selection of advice items from which the dental professional can select one or more.

The secondary user enters raw findings into a digital patient record or into the software application using their computing device.

An example of the raw findings of the secondary user might be the amount of plaque/calculus detected per tooth.

An algorithm 44 encoded in the software application analyses the raw findings 54 and automatically turns these findings into one or more advice items 62 from a limited set of predefined advice items concerning how to improve oral cleaning. This results in automation of the step of converting raw findings (i.e. simple assessment of the teeth) to the selection of appropriate advice for improving of the brushing.

Two concrete examples are as follows.

The raw findings might indicate accumulation of plaque/calculus on the outside of several upper molars on both sides of the jaw. The advice generation algorithm 44 converts this into the predefined qualitative advice item 62: "longer brushing of the outside of the upper molars teeth". Compliance can be later monitored by comparing the duration of the brushing by the primary user of the outside of the upper molars with the current/latest brushing session. In this regard, the advice item may additionally include an encoded representation of acceptable values for the cleaning parameter 76 'duration of brushing of the outside of the upper molars'. This cleaning parameter can be measured in a cleaning session using a simple timing module and using the IMU (or other sensor) to track positioning of the toothbrush head in the mouth over time, so that time spent in the relevant region of the mouth can be measured and recorded.

As a second example, the raw findings might indicate retraction of the gum in the upper right teeth. The advice generation algorithm 44 converts this into the predefined qualitative advice: "more gentle brushing of the upper right teeth". Compliance can be monitored by comparing the amount of pressure applied, and the duration of the brushing of the upper right teeth, with the current/latest brushing session. In this regard, the advice item may additionally include an encoded representation of acceptable values for the cleaning parameter 76 'amount of pressure applied, and the duration of the brushing of the upper right teeth'. Thus could for instance be an area under the curve of pressure vs time for the relevant location, or another parameter representation. This cleaning parameter can be measured in a cleaning session using a simple timing module, using the IMU (or other sensor) to track positioning of the toothbrush head in the mouth over time, so that time spent in the relevant region of the mouth can be measured and recorded, and using a pressure sensor which records brushing pressure.

Thus, from this example can be drawn a principle of more general application to any embodiment, namely, that it is particularly advantageous if each advice item 62 generated by the advice algorithm 44 includes qualitative, preferably linguistically expressed, advice which can be communicated to a user with a user-perceptible output, such as a vocal output or display output or otherwise, in combination with a corresponding encoded quantitative value for one or more oral cleaning parameters 76 which map on to the qualitative advice and permit compliance with the advice to be tested by acquiring sensor data 74 to measure the oral cleaning parameter in practice. This offers the best combination of advice: verbal advice for the user indicating a particular change to cleaning technique, in combination with computer-encoded parameters which permit testing of compliance with the advice by acquisition of the sensor data 74.

Returning to the particular set of embodiments being discussed previously, in some embodiments, the automatically generated advice 62 maybe fed back to the dentist and the dentist may be asked to confirm/refine/reject the advice before the advice is stored in the database 32. This extra check can reduce the chance of mistakes, improves transparency of the system, and ensures the dentist remains in full control.

The task of this automatic conversion 44 from raw findings 54 into advice 62 reduces the set of possible advice in such a way that identification of the most optimal advice becomes easier and more efficient.

Continuing description of the process flow, after the patient returns home and starts using the toothbrush, this event (the activation of the toothbrush) may be automatically sensed, e.g. by the IMU sensor. A link may be automatically established between the toothbrush and a computing device installed with the software application, adapted to implement the second part 10b of the method already described. The application contacts the database 32 and searches for any new advice which has been added. For any new advice items 62 found, the mobile device or toothbrush may play a vocalization of the advice to the user as a reminder (or any other sensory representation of the advice). This can be done one time, several times, or e.g. until the user indicates the reminder is no longer wanted. In some cases, the communication of the advice to the user is done by the toothbrush device (e.g. using an LED, or by audio means.

The compliance of the user brushing behavior with the advice is monitored. As discussed above, this is based on measuring sensor data, e.g. based on the readout of the brushing pressure sensor in combination with brush head localization. If the comparison step 78 (discussed previously) indicates that the cleaning parameters of the brushing session are not (if comparison is done in real time) or was not (if comparison is done after the event) in accordance with the advice 62 of the dentist, a warning may be given (e.g. audio, light, a written message displayed on a screen of the computing device carrying the software application). If it is/was in accordance, no feedback, or positive feedback may be given to the user. Non-compliance may result in extension of the period for which the user is reminded of the relevant advice of the dentist.

In an extension to the previous embodiment, the raw findings 54 that are used as input to the advice generation algorithm 22 could be combined with data from previously recorded brushing sessions of the user.

In some embodiments the compliance monitoring data may additionally be shared with a medical insurance company of the user. Compliance could for instance result in a discount on insurance fees, to incentivize compliance.

From the result 80 of the comparison, the dentist may refine their advice, or decide on new advice for how to improve cleaning habits.

Further to the already described process in which raw findings 54 are automatically converted into advice items 62, additionally the dentist (secondary user 52) may manually input advice. This may be selected by the secondary user from a limited set of predefined advice items, by selecting the one which is the closest match to the advice that they want to communicate. The advice options could be presented in a pull-down menu with a limited set of predefined advice items. Optionally, the advice options may be sorted according to relevancy for the case at hand. Relevancy could for example be determined or defined by statistics of previous cases, or could be determined by a trained AI algorithm, or could be derived by any other means known to a person skilled in the art.

It can also be entered into the digital patient record of the patient, and then uploaded from the digital patient record into the central advice database 32. It may be directly communicated to a mobile computing device of the user, after identifying the mobile phone and after establishing a private secure connection. After uploading, the connection is closed.

In a similar manner to above, with regards to input of the raw findings 52, a dedicated graphical user interface may be presented at the input device at the first location used by the secondary user. The graphical user interface may define discrete input fields, each corresponding to one of the standardized oral parameters, and wherein the oral examination findings are received as user input to the graphical user interface.

Embodiments of this invention thus provide a seamless tele-dentistry system, including technology for: user data analysis, for facilitating communication between dentists and patients, intelligent user behavior analysis, for the diagnosis of oral health status, for the automated generation of advice concerning how to improve or change daily oral cleaning behavior, including the generating of reminders of the advice, and for the assessment of compliance with the advice.

Another aspect of the invention is a computer program product comprising code means configured when executed by a processor to perform either one of two methods according to a selected mode,
wherein the method according to a first mode comprises:
   receiving as user input from the secondary user at an input device oral examination findings corresponding to the primary user, wherein the findings comprise user-input values for a pre-defined set of standardized oral parameters which together define an examination finding set;
   applying an advice generation algorithm adapted to convert an examination finding set to oral care advice comprising one or more oral care advice items, the advice generation algorithm defining mappings between different possible values for the pre-defined set of standardized oral parameters and output advice items;
   uploading the oral care advice to a record in a central user database, the record associated with the identity of the primary user;
   wherein each oral care advice item includes an encoded representation of acceptable parameter values for one or more oral cleaning parameters; and
wherein the method according to a second mode comprises:
   receiving sensor data obtained by an oral care device during one or more oral cleaning sessions of the primary user from sensors integrated in the oral care device,
   applying a sensor data conversion algorithm for converting the sensor data to an estimation of values for the one or more oral cleaning parameters for at least one oral cleaning session,
   comparing the estimated values for the one or more oral cleaning parameters with the acceptable parameter values encoded in the oral care advice, and
   generating an output based on the comparison.

For example, the first mode may be implemented when the application is run on a user-input device at a dentist's office.

For example, the second mode might be implemented when the application is run on a mobile computing device of the primary user communicatively linked with the oral care device, or by the oral care device itself.

Embodiments of the invention described above employ one or more processors. A processor may in general comprise a single processor or a plurality of processors. It may be located in a single containing device, structure, or unit, or it may be distributed between a plurality of different devices, structures or units. Reference therefore to the processor being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method (10) for facilitating interaction between a primary user and a secondary user of an oral care system, wherein the primary user is a user of an oral care device and the secondary user is a dental care professional, the method comprising:
receiving (12) as user input from the secondary user, at an input device at a first location, oral examination findings corresponding to the primary user, wherein the findings comprise user-input values for a pre-defined set of standardized oral parameters which together define an examination finding set;
applying (14) an advice generation algorithm adapted to convert an examination finding set to oral care advice comprising one or more oral care advice items, the advice generation algorithm defining mappings between different possible values for the pre-defined set of standardized oral parameters and output oral care advice items;
uploading (16) the oral care advice to a record in a user database, the record associated with the identity of the primary user;
wherein each oral care advice item includes an encoded representation of acceptable parameter values for one or more oral cleaning parameters; and
wherein the method further comprises, at a second location:
an oral care device obtaining (20) sensor data during one or more oral cleaning sessions of the primary user from sensors integrated in the oral care device,
applying (22) a sensor data conversion algorithm for converting the sensor data to an estimation of values for the one or more oral cleaning parameters for at least one oral cleaning session,
comparing (24) the estimated values for the one or more oral cleaning parameters with the acceptable parameter values encoded in the oral care advice, and
generating (26) an output based on the comparison.

2. The method of claim 1, wherein application of the sensor data conversion algorithm and/or the comparing of the estimated values for the cleaning parameters is performed by a processor of the oral care device or by a processor of a computing device communicably linked with the oral care device.

3. The method of any of claims 1-2, wherein the one or more oral care advice items output from the advice generation algorithm are presented to the secondary user via a user interface, and wherein the secondary user is prompted to confirm, reject, or refine the one or more advice items using the user interface.

4. The method of any of claims 1-3, wherein the method further comprises, responsive to a user-activation of the oral care device, transmitting a notification signal to a computing device, the computing device establishing communication with the user database responsive to the activation signal and downloading the oral care advice and generating a user-perceptible output at the computing device or the oral care device indicative of the oral care advice.

5. The method of any of any of claims 1-4, wherein the oral care device is a powered toothbrush, and the sensors include one or more of: pressure sensors for sensing brushing pressure, an inertial measurement unit for use in determining a pose of the oral care device, and one or more optical sensors for optical interaction with oral surfaces during use of the device.

6. The method of any of claims 1-5, wherein the method comprises presenting at said input device at the first location a graphical user interface, the graphical user interface defining discrete input fields, each corresponding to one of the standardized oral parameters, and wherein the oral examination findings are received as user input to the graphical user interface.

7. The method of any of claims 1-6, wherein the method comprises generating a user compliance report based on the comparison, indicative of a degree of compliance of the primary user with the oral care advice and to transmit the compliance report to a further device as a data package.

8. The method of claim 7, wherein the method further comprises uploading the compliance report to a server and storing it at the server in such a way as to be accessible by the secondary user via a computing device communicatively linked to the server.

9. The method of any of claims 1-8, wherein, following the generating of the oral care advice, the oral care advice is uploaded to a digital patient record associated with the primary user in a patient record database.

10. The method of any of claims 1-9, wherein the output is a user-perceptible output indicative of the oral care advice, and optionally wherein the user-perceptible output is generated by the oral care device, e.g. by illumination of a subset of one or more LEDs comprised by the device, to provide a coded indication of the advice, or by control of an acoustic output device of the oral care device to play a spoken-language indication of the advice.

11. The method of any of claims 1-10, wherein the one or more oral care advice items output from the advice generation algorithm are items from a pre-determined list of oral care advice items.

12. The method of any of claims 1-11, wherein the advice generation algorithm comprises a lookup table which maps between findings and advice items.

13. A computer program product comprising code means configured when executed by a processor to perform either one of two methods according to a selected mode,
wherein the method according to a first mode comprises:
receiving as user input from the secondary user at an input device oral examination findings corresponding to the primary user, wherein the findings comprise user-input values for a pre-defined set of standardized oral parameters which together define an examination finding set;
applying an advice generation algorithm adapted to convert an examination finding set to oral care advice comprising one or more oral care advice items, the advice generation algorithm defining mappings between different possible values for the pre-defined set of standardized oral parameters and output advice items;
uploading the oral care advice to a record in a user database, the record associated with the identity of the primary user;
wherein each oral care advice item includes an encoded representation of acceptable parameter values for one or more oral cleaning parameters; and
wherein the method according to a second mode comprises:
receiving sensor data obtained by an oral care device during one or more oral cleaning sessions of the primary user from sensors integrated in the oral care device,
applying a sensor data conversion algorithm for converting the sensor data to an estimation of values for the one or more oral cleaning parameters for at least one oral cleaning session,
comparing the estimated values for the one or more oral cleaning parameters with the acceptable parameter values encoded in the oral care advice, and
generating an output based on the comparison.

14. A system (50) for use in facilitating interaction between a primary user (66) and a secondary user (52), wherein the primary user is a user of an oral care device (70) and the secondary user is a dental care professional, the system comprising:
a first device (72) at a first location comprising one or more processors and adapted to:
receive as user input from a secondary user at an input device (42) oral examination findings (54) corresponding to the primary user (66), wherein the findings comprise user-input values for a pre-defined set of standardized oral parameters which together define an examination finding set;
apply an advice generation algorithm (44) adapted to convert an examination finding set to oral care advice comprising one or more oral care advice items (62), the advice generation algorithm defining mappings between different possible values for the pre-defined set of standardized oral parameters to output oral care advice items;
upload the oral care advice to a record in a user database (32), the record associated with the identity of the primary user (66);
wherein each oral care advice item includes an encoded representation of acceptable parameter values for one or more oral cleaning parameters (76); and
a second device (72), comprising one or more processors adapted to:
receive sensor data (74) obtained by an oral care device (70) during one or more oral cleaning sessions of the primary user (66) from sensors integrated in the oral care device,
apply a sensor data conversion algorithm (75) for converting the sensor data to an estimation of values for the one or more oral cleaning parameters (76) for at least one oral cleaning session,
compare (78) the estimated values for the one or more oral cleaning parameters with the acceptable parameter values encoded in the oral care advice, and
generate an output (80) based on the comparison.

15. The system of claim 14, wherein the second device is the oral care device or is a mobile computing device communicatively linked with the oral care device.
